# EUROPEAN PATENT SPECIFICATION

(11) **EP 1 531 684 B1**
(45) Date of publication and mention of the grant of the patent: **17.10.2007**
(21) Application number: 03785297.7
(22) Date of filing: 08.08.2003
(51) Int. Cl.: A23K 1/16, A23K 1/18, A23K 1/14

(54) **METHOD FOR CONTROLLING HAIRBALLS**
VERFAHREN ZUR KONTROLLE VON HAARBALLEN
PROCEDE POUR TRAITER LA FORMATION DE BOULES DE POILS

(30) Priority: 13.08.2002 US 403107 P
(43) Date of publication of application: 25.05.2005
(73) Proprietor: THE IAMS COMPANY, Dayton, Ohio 45414 (US)
(72) Inventor: DAVENPORT, Gary, Mitchell, Dayton, OH 45415 (US); MINIKHIEM, Debbie, Lee, Eaton, OH 45320 (US)
(74) Representative: Clemo, Nicholas Graham
(86) International application number: PCT/US2003/025797
(87) International publication number: WO 2004/014147

(56) References cited:
- WO-A-01/50881
- US-A- 6 080 403
- MILLER K W ET AL: "A 20-month olestra feeding study in dogs." FOOD AND CHEMICAL TOXICOLOGY 1991 CORRESPONDENCE (REPRINT) ADDRESS, K. D. LAWSON, PROCTER & GAMBLE CO., 6071 CENTER HILL RD., CINCINNATI, OH 45224-1703, USA, vol. 29, no. 7, 1991, page 427, XP0009021077

## Description

### FIELD OF THE INVENTION

The present invention relates to methods of treating a hairball or increasing fecal hair excretion in a mammal in need of such treatment through use of a polyol fatty acid polyester. The invention further relates to veterinary or feed compositions comprising a polyol fatty acid polyester.

### BACKGROUND OF THE INVENTION

Hairballs (trichobezoar) can be prevalent in some mammals, such as cats and rabbits, because of natural grooming habits. Ingested hair that is not digested or that does not otherwise pass through the digestive tract accumulates in the stomach and can form a hairball.

Common physiological consequences of hairballs include diarrhea, vomiting, constipation and other difficulties if the hairball becomes lodged in the mammal's lower bowel. Such obstructions can become life-threatening and require surgical intervention.

Conventional methods of treating hairballs include the administration of lubricants (*e*.*g*., petroleum jelly or mineral oil), or pineapple juice (see, for example, U.S. Patent No. 6,080,403), as well as feeding diets high in insoluble fiber. However, these methods may be limited in their effectiveness, convenience, or by their associated side effects.

Thus, there remains a need for additional methods for treating hairballs and for increasing fecal hair excretion.

### SUMMARY OF THE INVENTION

The present invention is directed to the use of compositions comprising a polyol fatty acid polyester. These compositions, whether the polyol fatty acid polyester is alone or in combination with one or more other components (*e*.*g*., a dietary fiber source), are useful for treating hairballs and for increasing fecal hair excretion.

Accordingly, the invention provides methods of treating hairballs in a mammal in need of such treatment comprising administering to the mammal an effective amount of a polyol fatty acid polyester.

The invention also provides a method for increasing fecal hair excretion in a mammal in need of such treatment comprising administering to the mammal an effective amount of a polyol fatty acid polyester.

### DETAILED DESCRIPTION OF THE INVENTION

The present invention is directed to methods selected from treating hairballs, increasing fecal hair excretion, and combinations thereof comprising administering to a mammal in need thereof a composition comprising a polyol fatty acid polyester (herein often referenced as a "PFAP"). The invention also provides a veterinarian or pharmaceutical composition comprising an amount of a polyol fatty acid polyester effective to increase fecal hair excretion or to treat a hairball in a mammal.

As used herein, methods of treating hairballs include methods of ameliorating at least one symptom of, curing, alleviating, inhibiting, preventing, or decreasing the frequency of the development of, a hairball, or any combination thereof. Any statistically significant attenuation of one or more symptoms associated with a hairball is considered to be a treatment within the scope of this invention.

As such, for example, the present methods may minimize the formation of hairballs in a mammal, decrease the frequency thereof or alleviate one or more of the associated undesirable effects associated therewith.

All mammals are included for treatment herein. In a preferred embodiment, the mammal is any mammal which experiences, or is susceptible to, hairballs. Such mammals may include, for example, cattle, cats, rats, rabbits, primates, and humans. Preferred mammals for treatment herein are cats.

As also used herein, the term "administering" with regard to the mammal means to provide the referenced composition to such mammal and/or to direct, instruct, or advise the administration of the composition for a purpose described herein. Wherein the administration of the composition is directed, instructed or advised, such direction may be that which instructs and/or informs the user or purchaser, or any other individual (including, for example, the owner of the mammal treated herein), that administration of the composition may and/or will provide one or more of the benefits describe herein, for example, treatment of hairballs or increased fecal hair excretion.

Administration which is directed may comprise, for example, oral direction (*e*.*g*., through oral instruction from, for example, a veterinarian or other health professional, sales professional or organization, and/or radio or television media (*i*.*e*., advertisement) or written direction (*e*.*g*., through written direction from, for example, a veterinarian or other health professional (*e*.*g*., scripts), sales professional or organization (*e*.*g*., through, for example, marketing brochures, pamphlets, or other instructive paraphernalia), written media (*e*.*g*., internet, electronic mail, or other computer-related media), and/or packaging associated with the composition (*e*.*g*., a label present on a package containing the composition). As used herein, "written" includes through words, pictures, symbols, and/or other visible descriptors. Such direction need not utilize the actual words used herein, but rather use of words, pictures or symbols conveying the same or similar meaning are contemplated within the scope of this invention.

The present methods comprise administration of a composition comprising a polyol fatty acid polyester. Compositions utilized in accordance with the present inventive methods include any polyol fatty acid polyester that, when administered to the mammal, increases fecal hair excretion or treats hairball in a mammal (as defined herein above). The compositions utilized in accordance with the present methods are described further, as follows:

As used herein, the term "polyol," includes any aliphatic or aromatic compound containing at least two free hydroxyl groups. Such polyols will be well-known in the art. In accordance with the present invention, the selection of a suitable polyol is simply a matter of choice. For example, suitable polyols may be selected from the following classes: saturated and unsaturated straight and branched chain linear aliphatic; saturated and unsaturated cyclic aliphatic, including heterocyclic aliphatic; or mononuclear or polynuclear aromatics, including heterocyclic aromatics. Carbohydrates and glycols are exemplary polyols. Monosaccharides suitable for use include, for example, mannose, galactose, arabinose, xylose, ribose, apiose, rhamnose, psicose, fructose, sorbose, tagitose, ribulose, xylulose, and erythrulose. Oligosaccharides suitable for use include, for example, maltose, kojibiose, nigerose, cellobiose, lactose, melibiose, gentiobiose, turanose, rutinose, trehalose, sucrose and raffinose. Polysaccharides suitable for use include, for example, amylose, glycogen, cellulose, chitin, inulin, agarose, zylans, mannan and galactans. Although sugar alcohols are not carbohydrates in a strict sense, the naturally occurring sugar alcohols are so closely related to the carbohydrates that they are also preferred for use herein. The sugar alcohols most widely distributed in nature and suitable for use herein are sorbitol, mannitol and galactitol.

Particular polyols suitable for use herein include monosaccharides, disaccharides and sugar alcohols. Other classes of materials include sugar ethers and alkoxylated polyols, such as polyethoxy glycerol.

In one embodiment of the present invention the polyol has on average at least 4, preferably at least about 5, more preferably about 8 hydroxyl groups capable of being esterified per polyol molecule.

Suitable esterified epoxide-extended polyols include esterified propoxylated glycerols prepared by reacting a propoxylated glycerol having from 2 to 100 oxypropylene units per glycerol with fatty acids (*e*.*g*., C₁₀-C₂₄ fatty acids) or with fatty acid esters (*e*.*g*., C ₁₀-C₂₄ fatty acid esters), as described in U.S. Patent Nos. 4,983,329 and 5,175,323, respectively, and esterified propoxylated glycerols prepared by reacting an epoxide and a triglyceride with an aliphatic polyalcohol, as described in U.S. Patent No. 5,304,665 or with an alkali metal or alkaline earth salt of an aliphatic alcohol, as described in U.S. Patent No. 5,399,728. Other polyols include acylated propylene oxide-extended glycerols having a propoxylation index of above about 2, preferably in the range of from about 2 to about 8, more preferably about 5 or above, wherein the acyl groups are preferably C₈-C_{24,} and more preferably C₁₄-C₁₈, compounds, as described in U.S. Patent Nos. 5,603,978 and 5,641,534 and fatty acid-esterified propoxylated glycerols, as described in U.S. Patent Nos. 5,589,217 and 5,597,605.

Other suitable esterified epoxide-extended polyols include esterified alkoxylated polysaccharides. Preferred esterified alkoxylated polysaccharides are esterified alkoxylated polysaccharides containing anhydromonosaccharide units, more preferred are esterified propoxylated polysaccharides containing anhydromonosaccharide units, as described in U.S. Patent No. 5,273,772.

The polyol fatty acid polyester includes esters of a polyol with a degree of esterification greater than the degree of esterification of the polyol. In one embodiment of the invention the polyol fatty acid polyester has a degree of esterification of at least about 70%, while in yet another embodiment the polyol fatty acid polyester has a degree of esterification of at least about 90%, preferably at least about 95%.

A variety of processes are known in the art for the synthesis of polyol fatty acid polyesters that are suitable for use in the processes of the present invention. Examples of such processes are detailed in U.S. Patent No. 3,963,699, disclosing a solvent-free transesterification process in which a mixture of a polyol (such as sucrose), a fatty acid lower alkyl ester (such as a fatty acid methyl ester), an alkali metal fatty acid soap, and a basic catalyst is heated to form a homogenous melt. Excess fatty acid lower alkyl ester is added to the melt to form the higher polyol fatty acid polyesters. The polyesters are then separated from the reaction mixture by any of the routinely used separation procedures; distillation or solvent extraction are preferred. Additional suitable processes include those disclosed in U.S. Patent Nos. 4,517,360; 5,422,131; 5,648,483; 5,767,257; and 6,261,628.

In one embodiment of the present invention, the polyol fatty acid polyesters are sucrose fatty acid polyesters, having an average of at least 4 fatty acid groups per molecule. In another embodiment of the invention, the polyol fatty acid polyester is sucrose fatty acid polyester having an average of at least 5 fatty acid groups per molecule, while in another embodiment the sucrose fatty acid polyesters have an average of from about 5 to about 8 fatty acid groups per molecule. In yet another embodiment, the polyol polyester is a sucrose polyester wherein at least about 75% of the sucrose polyester comprises octaester.

The fatty acid chains of the polyol fatty acid polyesters may be branched, linear, saturated, unsaturated, hydrogenated, unhydrogenated, or mixtures thereof. The fatty acid chains of the fatty acid esters typically have from about 2 to about 30 total carbon atoms. As used herein, reference to a fatty acid compound having fatty acid chains of a particular length is intended to mean that a majority of the fatty acid chains, i.e., greater than 50 mol % of the fatty acid chains, have the stated length. In a more specific embodiment, the fatty acid compounds have greater than about 60 mol %, and more specifically greater than about 75 mol %, of fatty acid chains of the stated length.

Other suitable polyol fatty acid polyesters are esterified linked alkoxylated glycerins, including those comprising polyether glycol linking segments, as described in U.S. Patent No. 5,374,446 and those comprising polycarboxylate linking segments, as described in U.S. Patent Nos. 5,427,815 and 5,516,544.

Additional suitable polyol fatty acid polyesters are esterified epoxide-extended polyols of the general formula P(OH)_{A+C} (EPO)_{N} (FE)_{B} wherein P(OH) is a polyol, A is from 2 to about 8 primary hydroxyls, C is from about 0 to about 8 total secondary and tertiary hydroxyls, A + C is from about 3 to about 8, EPO is a C₃-C₆ epoxide, N is a minimum epoxylation index average number, FE is a fatty acid acyl moiety and B is an average number in the range of greater than 2 and no greater than A + C, as described in U.S. Patent No. 4,861,613. The minimum epoxylation index average number has a value generally equal to or greater than A and is a number sufficient so that greater than 95% of the primary hydroxyls of the polyol are converted to secondary or tertiary hydroxyls. Preferably the fatty acid acyl moiety has a C₇-C₂₃ alkyl chain.

The polyol fatty acid polyester may be comprised of a single type or class of polyol polyester (*e*.*g*., sucrose) or may alternatively be a blend of two or more types or classes ofpolyol polyesters (*e*.*g*., sugar alcohols, such as sorbitol; monosaccharides, such as fructose; and oligosaccharides, such as maltose). The polyol backbones of the polyol fatty acid polyesters (e.g., sucrose in a sucrose fatty acid polyester) may be the same backbone as the polyol, or may optionally be comprised of two or more different polyol backbones.

In one embodiment of the present invention the polyol is sucrose and the polyol fatty acid polyester is predominantly (i.e., in excess of about 95%, preferably in excess of about 98%, more preferably in excess of about 99%) comprised of sucrose fatty acid polyester. In another embodiment the polyol is glucose and the polyol fatty acid polyester is sucrose fatty acid polyester. In another embodiment, the polyol is glucose and the polyol fatty acid polyester is glucose fatty acid polyester. In yet another embodiment, the polyol is sucrose and the polyol fatty acid polyester is comprised of sucrose fatty acid polyester and esterified epoxide-extended polyol polyester.

As used herein "fatty acid ester" includes fatty acid esters in which the fatty acid chains have a total of from about 2 to about 28, typically from about 8 to about 22, carbon atoms. The fatty acid esters may be branched, unbranched, saturated, unsaturated, hydrogenated, unhydrogenated, or mixtures thereof. In one embodiment of the present invention, the fatty acid chains of the polyester may be branched or linear and may be formed from fatty acid esters having fatty acid chains of from about 8 to about 26 total carbon atoms. In yet another embodiment, the fatty acid chains of the fatty acid ester have from about 16 to about 22 total carbon atoms.

Examples of suitable fatty acids include caprylic, capric, lauric, myristic, myristoleic, palmitic, palmitoleic, stearic, oleic, linoleic, eleostearic, arachidic, behenic and erucic. Many fatty acids or oils are commercially available or can be readily prepared or isolated using procedures known to those skilled in the art. Many commercially available fatty acids and laboratory prepared fatty acids, depending upon their method of preparation, for example, by saponification of triglycerides or animal fat, can contain mixtures of fatty acids which have a distribution of different chain lengths, with or without unsaturation, and with or without positional or geometric isomers.

The term "fatty acid" encompasses fatty acid, fatty oils, or mixtures thereof as conventionally defined, for example, long-chain aliphatic acids found in natural fats and oils. The term "fatty acid" also encompasses similar or chemically related compounds which include a fatty or oil soluble portion, such as a C₂₋₂₈ aliphatic chain and an acid group, such as a carboxylate, sulfonate, phosphate or phosphonate. The "fatty acid" can be prepared synthetically such as known synthetic emulsifiers, surfactants or tensides. The "fatty acid" compounds are capable of "esterifying" or forming an equivalent chemical linkage with a polyol and the resulting polyol fatty acid polyesters are capable of modifying the surface properties of hairball masses and hairball constituents.

Studies have shown that human consumption of certain PFAP's is safe with minima side effects (Thomson et al., Aliment. Pharmacol. Ther., Vol. 12, 1185-1200 (1998)). Typically, PFAP's are not digested or absorbed by the body because gastric and pancreatic lipases do not hydrolyze the ester bond linking the fatty acids to the polyol (Jandacek et al., Lipids, Vol. 34, 771-783 (1999)). They are not absorbed from the small intestine, and as a result are excreted in the feces in their intact form, and thus may be used as fat replacers in low-fat food products.

A 20-month feeding study involving dogs showed that diets containing 5 and 10% of a representative PFAP (a mixture of 99% hepta- and octaesters of sucrose and long chain fatty acids) did not produce any undesirable side effects compared with a PFAP-free diet (Miller et al., Fd. Chem. Toxic., Vol. 29, 427-435 (1991)). From the study, it was concluded that the PFAP was not toxic to dogs when fed up to 10% of the diet based on growth characteristics, survivability, hematology and histopathology. Miller *et al*. report that voluntary feed consumption was higher for the PFAP-fed dogs compared with controls, and also report isolated incidences of soft stools.

Furthermore, results of a 26-week feeding study showed that the inclusion of up to 5% of the same representative PFAP as above in the diet of weanling pigs did not affect digestible feed energy or overall growth (Cooper et al., J. Nutr., 1997, 127, 1609S-1635S).

A polyol fatty acid polyester utilized in accordance with the present invention can be formulated as a food, pharmaceutical, or veterinary composition and administered to a mammal, such as a cat or rabbit, in a variety of forms adapted to a chosen route of administration, for example, orally, parenterally, intravenously or subcutaneously. A preferred method of administration is oral administration. For example, the polyol fatty acid polyester can be administered orally by incorporating the polyol fatty acid polyester and optionally an excipient or carrier into the mammal's diet, such as mixed with the mammal's food or feed ration, or as a dietary supplement, such as pill, pellet, biscuit or elixir.

Optionally, the compositions utilized in accordance with the present inventive method may further include one or more optional components which further assist with the hairball and fecal hair excretion benefits described herein or provide one or more other benefits. For example, the compositions used herein may comprise one or more dietary fibers to enhance the hairball and fecal hair excretion benefits herein. Non-limiting examples of such dietary fibers include cellulose, carboxymethylcellulose, soybean (including for example soybean hulls), rice (including for example rice hulls and rice bran), pea fiber, beet pulp and mixtures thereof. As a further example, the compositions utilized herein can be systemically administered in combination with a veterinary acceptable vehicle such as an inert diluent, or an assimilable edible carrier. Hard- or soft-shell gelatin capsules or compressed tablets may be utilized, or the PFAP maybe incorporated directly with the food of the mammal's diet. For oral administration, the polyol fatty acid polyester can be combined with one or more excipients and used in the form of ingestible tablets, buccal tablets, troches, capsules, elixirs, suspensions, syrups or wafers.

Such compositions and preparations typically contain at least 0.05% polyol fatty acid polyester, or alternatively at least 0.1% polyol fatty acid polyester, each by weight of the composition. Alternatively or additionally, the compositions may conveniently comprise from about 0.1% to about 99% PFAP, or from about 1% to about 60% PFAP, each by weight of the composition. The amount of PFAP in such therapeutically useful compositions is such that an effective dosage level will be obtained.

The compositions of the invention are preferably administered to treat hairballs or to increase fecal hair excretion in a mammal. To achieve such effects, the compositions may be administered such that PFAP dosages of from about 0.001 to about 400 mg/kg, more preferably from about 0.01 to about 200 mg/kg, and even more preferably from about 0.1 to about 175 mg/kg, are delivered based on the body weight of the mammal, although other dosages may provide beneficial results. For example, alternatively or additionally, a suitable dose of PFAP in the composition maybe in the range of from about 0.5 to about 400 mg/kg, from about 50 to about 300 mg/kg, from about 50 to about 250 mg/kg, from about 50 to 200 mg/kg, or from about 100 to 175 mg/kg based on the body weight of the mammal. These doses may optionally be daily doses.

The amount of PFAP administered will vary depending on various factors including, but not limited to, the type, the particular disease or condition, the weight, the physical condition of the mammal, the age of the mammal, whether prevention or treatment is to be achieved and the route of administration. Such factors may be readily determined by clinicians, veterinarians, or pet food manufacturers.

Administration in accordance with the present invention may be continuous or intermittent, depending, for example, upon the recipient's physiological condition, whether the purpose of the administration is therapeutic or prophylactic, and other factors known to skilled practitioners. The administration of the agents of the invention may be essentially continuous over a preselected period of time or may be in a series of spaced doses. For example, the desired dose may conveniently be presented in a single dose or as divided doses administered at appropriate intervals, *e*.*g*., as two, three, four or more sub-doses per day. The sub-dose itself may be further divided, *e*.*g*., into a number of discrete loosely spaced administrations, such as multiple servings of a high-quality food matrix.

Compositions containing the PFAP can be prepared by procedures known in the art using known and readily available ingredients. For example, the agent can be formulated with common excipients, diluents, or carriers, and formed into tablets, capsules, suspensions or powders. Examples of excipients, diluents, and carriers that are suitable for such formulations include the following fillers and extenders such as starch, sugars, mannitol, and silicic derivatives; binding agents such as carboxymethyl cellulose, HPMC and other cellulose derivatives, alginates, gelatin, and polyvinyl-pyrrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as cetyl alcohol, glycerol monostearate; absorptive carriers such as kaolin and bentonite; and lubricants such as talc, calcium and magnesium stearate, and solid polyethyl glycols.

For example, tablets or caplets containing the agents of the invention can include buffering agents such as calcium carbonate, magnesium oxide and magnesium carbonate. Caplets and tablets can also include inactive ingredients such as cellulose, pregelatinized starch, silicon dioxide, hydroxy propyl methyl cellulose, magnesium stearate, microcrystalline cellulose, starch, talc, titanium dioxide, benzoic acid, citric acid, corn starch, mineral oil, polypropylene glycol, sodium phosphate, and zinc stearate. Hard or soft gelatin capsules containing an agent of the invention can contain inactive ingredients such as gelatin, microcrystalline cellulose, sodium lauryl sulfate, starch, talc, and titanium dioxide, as well as liquid vehicles such as polyethylene glycols (PEGs) and vegetable oil. Moreover, enteric coated caplets or tablets of an agent of the invention are designed to resist disintegration in the stomach and dissolve in the more neutral to alkaline environment of the duodenum.

The therapeutic agents of the invention can also be formulated as elixirs or solutions for convenient oral administration.

Food compositions utilized in accordance with the present invention are typically nutritionally balanced mixtures and include, but are not limited to, high-moisture (greater than about 50% moisture) foods, kibbles (including dry or low-moisture content pet foods (less than about 15% moisture)), and semi-dry pet foods (from about 15% to about 50% moisture). The food compositions described herein are not limited to a specific listing of ingredients because such ingredients will depend on such factors as, for example, the desired nutritional balance for the specific type of pet and availability of ingredients to the manufacturer. Cat food compositions are particularly preferred herein.

Typical formulae for pet food compositions are well known in the art. In addition to proteinaceous and farinaceous materials, the pet food compositions of the invention generally may include vitamins, minerals, and other additives such as flavorings, preservatives, emulsifiers and humectants. The nutritional balance, including the relative proportions of vitamins, minerals, protein, fat and carbohydrate, is determined according to dietary standards known in the veterinary and nutritional art. For example, the nutritional balance of a cat food composition is determined according to the known dietary requirements for cats. In addition to the proteinaceous and farinaceous materials, other materials such as whey and other dairy by-products, as well as other carbohydrates may be added. In addition, known flavorings including, for example, corn syrup or molasses, may be added.

The therapeutic agents of the invention may also be administered orally to a mammal *via* a complete food product or nutritional supplement. A food product or nutritional supplement of the invention is therapeutically effective in the treatment of hairballs and/or is effective to increase fecal hair excretion in an animal. A food product or nutritional supplement according to the invention can be artificially enriched in vitamins, soluble or insoluble mineral salts or mixtures thereof. It can also include one or more enzymes. A nutritional supplement of the invention can be formed as a concentrate with high level of a PFAP such that it requires dilution before feeding to the mammal. The supplement may be in any form, such as a non-liquid solid, powder, or semi-solid. A liquid form can be mixed in with food or fed directly to the mammal, for example via a spoon or *via* a pipette-like device.

The invention will now be illustrated by the following non-limiting examples.

### Example 1

The ability of a representative PFAP to increase fecal hair excretion and treat hairballs was evaluated.

PFAP was incorporated into a high-quality cat food matrix composition at a total level of 1.5% PFAP by weight of this composition (1.4% of a sucrose ester of a mixture of fatty acids derived from cotton seed oil, by weight of the composition, and 0.1 % of a sucrose ester ofbehenic acid, by weight of the composition).

A panel of 16 adult domestic cats were fed one of the following diets:

| | |
|---|---|
| Diet 1 | included carboxymethylcellulose (CMC) |
| Diet 2 | included PFAP |
| Diet 3 | included CMC and PFAP |
| Diet 4 | included cellulose |
| Diet 5 | included PFAP |
| Diet 6 | included cellulose and PFAP |

The cats were fed their respective diet during a 6-week experimental period that followed a 2-week baseline period. All cats received a commercially available cat food (Eukanuba® Chicken and Rice Cat Formula) during the baseline period. Measurements made during the 6-week experimental period included daily food consumption, daily stool scores, fecal hair excretion and overall diet digestibility.

### Results

**Table 1**

| Fecal Hair Excretion | Baseline Diet | | | Experimental Diet | | | Change | | Expt vs Baseline |
|---|---|---|---|---|---|---|---|---|---|
| | (Eukanuba Cat) | | | | | | | | |
| (g/day) | g | diet | time | g | diet | time | % | X | time |
| CMC | 0.08 | | | 0.09 | a | | 12.5 | 1.1 | NS |
| PFAP | 0.10 | | x | 0.22 | b | y | 120.0 | 2.2 | 0.13 |
| CMC + PFAP | 0.08 | | x | 0.26 | b | y | 225.0 | 3.3 | 0.01 |
| Diet (P<) Cellulose | 0.0259 | NS | | 0.0630 | 0.11 a | | 143.2 | 2.4 | |
| PFAP | 0.0250 | | | 0.0914 | a | | 265.6 | 3.7 | |
| Cellulose + PFAP | 0.1046 | | x | 0.5894 | b | y | 463.5 | 5.6 | 0.01 |
| Diet (P<) | | NS | | | 0.01 | | | | |

**Table 2**

| **Stool Score** | Baseline Diet | | | Experimental Diet | | | Change | | Expt vs Baseline |
|---|---|---|---|---|---|---|---|---|---|
| | (Eukanuba Cat) | | | | | | | | |
| | g | diet | time | g | diet | time | % | | time |
| CMC | 3.84 | | x | 3.19 | a | y | -16.9 | | 0.01 |
| PFAP | 4.13 | | | 3.89 | b | | -5.8 | | NS |
| CMC + PFAP | 3.96 | | x | 2.96 | a | y | -25.3 | | 0.01 |
| Diet (P<) Cellulose | 4.30 | NS a | | 4.15 | 0.06 | | -3.5 | | |
| PFAP | 4.30 | a | x | 4.03 | | y | -6.3 | | 0.07 |
| Cellulose + PFAP | 3.82 | b | | 4.01 | | | 5.0 | | |
| Diet (P<) | | 0.01 | | | NS | | | | |

**Table 3**

| Digestibility% | Dry matter | | Protein | | Fat | | Crude Fiber | | NFE | | DE | | ME | |
|---|---|---|---|---|---|---|---|---|---|---|---|---|---|---|
| CMC | 77.10 | | 79.98 | | 86.47 | a | 41.35 | | 81.70 | | 81.92 | | 75.06 | |
| PFAP | 81.77 | | 86.04 | | 86.50 | a | 29.61 | | 89.22 | | 86.19 | | 82.23 | |
| CMC +PFAP | 78.29 | | 82.99 | | 81.03 | b | 31.48 | | 84.74 | | 81.29 | | 76.61 | |
| Diet (P<) | | NS | | NS | | 0.10 | | NS | | NS | | NS | | NS |
| Cellulose | 80.38 | a | 87.14 | ab | 95.65 | a | 43.97 | | 73.00 | a | 85.94 | | 81.29 | |
| PFAP | 82.31 | ab | 86.88 | a | 87.35 | b | 40.23 | | 85.69 | b | 85.63 | | 81.26 | |
| Cellulose + PFAP | 83.33 | b | 89.07 | b | 91.54 | c | 48.45 | | 81.10 | c | 86.77 | | 82.38 | |
| Diet (P<) | | 0.07 | | 0.10 | | 0.01 | | NS | | 0.01 | | NS | | NS |

The inclusion of the PFAP in the cellulose- and CMC-based diet significantly increase fecal hair excretion with no detrimental effects on nutrient digestibility or subjective stool scores. The inclusion of PFAP in Diet 3 produced a two-fold increase in fecal hair excretion. In Diet 2, PFAP alone also increased fecal hair excretion by about 120% compared with baseline diets. The combination of cellulose and PFAP in Diet 6 produced about five times more fecal hair excretion than the baseline diet and about a two-fold increase compared with the Diet 4 and 5.

These results demonstrate that inclusion of PFAP only or PFAP in cellulose and CMC-based diets significantly increased fecal hair excretion. Moreover, there were no detrimental effects on nutrient digestibility or subjective stool scores.

### Example 2

The following illustrate representative: (i) veterinary dosage forms, and (ii) dietary or food supplement formulations, containing a PFAP for use in mammals. These compositons may be obtained by conventional procedures known in the veterinary and nutritional art.

| (i) Composition 1 | mg/day |
|---|---|
| PFAP | 100.0 |
| CMC | 75.0 |
| Other Fiber | 25.0 |
| | 200.0 |
| | |

| (ii) Composition 2 | mg/day |
|---|---|
| PFAP | 80.0 |
| Cellulose | 100.0 |
| Starch | 20.0 |
| | 200.0 |

All percentages and ratios are calculated by weight unless otherwise indicated. All percentages and ratios are calculated based on the total diet unless otherwise indicated.

Referenced herein are trade names for components including various ingredients utilized in the present invention. We do not intend to be limited by materials under a certain trade name. Equivalent materials (e.g., those obtained from a different source under a different name or reference number) to those referenced by trade name may be substituted and utilized in the descriptions herein.

In the description of the invention various embodiments and/or individual features are disclosed. As will be apparent to the ordinarily skilled practitioner, all combinations of such embodiments and features are possible and can result in preferred executions of the present invention.

The diets herein may comprise, consist essentially of, or consist of any of the elements as described herein.

## Claims

1. The use of a polyol fatty acid polyester in the manufacture of a composition for treating a hairball or increasing fecal hair excretion in mammals.

2. The use according to Claim 1 wherein the polyol fatty acid polyester is made from a polyol with at least 4 hydroxyl groups capable of being esterified.

3. The use according to any one of the preceding claims wherein the polyol fatty acid polyester comprises a polyol selected from monosaccharide, oligosaccharide or mixture thereof.

4. The use according to any of the preceding claims wherein the polyol fatty acid polyester has a degree of esterification of at least 70%.

5. The use according to any of the preceding claims wherein the polyol fatty acid polyester comprises one or more residues of caprylic, capric, lauric, myristic, myristoleic, palmitic, palmitoleic, stearic, oleic, linoleic, eleostearic, arachidic, behenic, or erucic acid.

6. The use according to any one of the preceding claims wherein the mammal is a cat or a rabbit.

7. The use according to Claim 6, wherein the mammal is a cat.

8. The use according to any one of the preceding claims wherein said composition comprises 0.05% of polyol fatty acid polyester, by weight of the composition.

9. The use according to any one of the preceding claims wherein said composition comprises one or more dietary fibers.

10. The use according to any one of the preceding claims wherein treating a hairball comprises ameliorating at least one symptom of curing, alleviating, inhibiting, preventing or decreasing the frequency of development of a hairball, or any combination thereof.

## Patentansprüche

1. Verwendung eines Polyolfettsäurepolyesters bei der Herstellung einer Zusammensetzung zur Behandlung eines Haarballs oder zur Erhöhung der Haarausscheidung mit dem Stuhl bei Säugern.

2. Verwendung nach Anspruch 1, wobei der Polyolfettsäurepolyester aus einem Polyol mit mindestens 4 Hydroxylgruppen, die veresterbar sind, besteht.

3. Verwendung nach einem der vorstehenden Ansprüche, wobei der Polyolfettsäurepolyester ein Polyol, ausgewählt aus Monosaccharid, Oligosaccharid oder Mischungen davon, umfasst.

4. Verwendung nach einem der vorstehenden Ansprüche, wobei der Polyolfettsäurepolyester einen Veresterungsgrad von mindestens 70 % aufweist.

5. Verwendung nach einem der vorstehenden Ansprüche, wobei der Polyolfettsäurepolyester einen oder mehrere Reste von Capryl-, Caprin-, Laurin-, Myristin-, Myristolein-, Palmitin-, Palmitolein-, Stearin-, Olein-, Linol-, Eleostearin-, Arachidin-, Behen- oder Erucasäure umfasst.

6. Verwendung nach einem der vorstehenden Ansprüche, wobei der Säuger eine Katze oder ein Kaninchen ist.

7. Verwendung nach Anspruch 6, wobei der Säuger eine Katze ist.

8. Verwendung nach einem der vorstehenden Ansprüche, wobei die Zusammensetzung zu 0,05 Gew.-% der Zusammensetzung Polyolfettsäurepolyester umfasst.

9. Verwendung nach einem der vorstehenden Ansprüche, wobei die Zusammensetzung einen oder mehrere Ballaststoffe Fasern umfasst.

10. Verwendung nach einem der vorstehenden Ansprüche, wobei die Behandlung eines Haarballs die Verbesserung mindestens eines Symptoms von Heilung, Linderung, Hemmung, Verhinderung oder Senkung der Häufigkeit der Entwicklung eines Haarballs oder eine beliebige Kombination davon umfasst.

## Revendications

1. Utilisation d'un polyester de polyol et d'acide gras dans la fabrication d'une composition pour le traitement du trichobézoard ou pour l'augmentation de l'excrétion fécale de poils chez les mammifères.

2. Utilisation selon la revendication 1, dans laquelle le polyester de polyol et d'acide gras est fabriqué à partir d'un polyol avec au moins 4 groupes hydroxyles susceptibles d'être estérifiés.

3. Utilisation selon l'une quelconque des revendications précédentes, dans laquelle le polyester de polyol et d'acide gras comprend un polyol choisi parmi le monosaccharide, l'oligosaccharide ou un de leurs mélanges.

4. Utilisation selon l'une quelconque des revendications précédentes, dans laquelle le polyester de polyol et d'acide gras a un degré d'estérification d'au moins 70 %.

5. Utilisation selon l'une quelconque des revendications précédentes, dans laquelle le polyester de polyol et d'acide gras comprend un ou plusieurs résidus d'acide caprylique, caprique, laurique, myristique, myristoléique, palmitique, palmitoléique, stéarique, oléique, linoléique, éléostéarique, arachidique, béhénique ou érucique.

6. Utilisation selon l'une quelconque des revendications précédentes, dans laquelle le mammifère est un chat ou un lapin.

7. Utilisation selon la revendication 6, dans laquelle le mammifère est un chat.

8. Utilisation selon l'une quelconque des revendications précédentes, dans laquelle ladite composition comprend 0,05 % de polyester de polyol et d'acide gras, en poids de la composition.

9. Utilisation selon l'une quelconque des revendications précédentes, dans laquelle ladite composition comprend une ou plusieurs fibres alimentaires.

10. Utilisation selon l'une quelconque des revendications précédentes, dans laquelle le traitement d'un trichobézoard comprend l'amélioration d'au moins un symptôme de durcissement, d'atténuation, d'inhibition, de prévention ou de diminution de la fréquence de développement d'un trichobézoard, ou n'importe laquelle de leurs combinaisons.
